# EUROPÄISCHE PATENTANMELDUNG

(11) **EP 3 797 831 A1**
(43) Veröffentlichungstag der Anmeldung: **31.03.2021**
(21) Anmeldenummer: 20198334.3
(22) Anmeldetag: 25.09.2020
(51) Int. Cl.: A61N 5/10

(54) **BRACHYTHERAPIEVORRICHTUNG**

(30) Priorität: 30.09.2019 DE 102019126326
(71) Anmelder: Carl Zeiss Meditec AG, 07745 Jena (DE)
(72) Erfinder: Röder, Norman, 99441 Döbritschen (DE)
(74) Vertreter: Diehl & Partner

(57) **Zusammenfassung**

Die vorliegende Erfindung betrifft eine Brachytherapievorrichtung (1) und ein Brachytherapieverfahren unter Verwendung der Brachytherapievorrichtung (1). Die Brachytherapievorrichtung (1) umfasst ein stabförmiges Hauptelement (3), eine erste Halterung (9), eine zweite Halterung (11), Nebenelemente (15), die sich zwischen der ersten Halterung (9) und der zweiten Halterung (11) erstecken und flexibel sind und mit der ersten Halterung (9) und der zweiten Halterung (11) so verbunden sind, dass eine Änderung der Lage der ersten Halterung (9) relativ zu der zweiten Halterung (11) eine Änderung eines Abstands zwischen dem Hauptelement (3) und dem mindestens einen Nebenelement (15) bewirkt; und eine Messvorrichtung (17), welche dazu konfiguriert ist, basierend auf mindestens einer Koordinate der Lage der ersten Halterung (9) relativ zu der zweiten Halterung (11) einen Messwert (w) anzugeben, welcher eine von dem Abstand abhängige Größe (D) repräsentiert.

## Beschreibung

Die vorliegende Erfindung betrifft eine Brachytherapievorrichtung und ein Brachytherapieverfahren, insbesondere ein elektronisches Brachytherapieverfahren.

Es existieren verschiedene etablierte Brachytherapieverfahren, welche mittels spezieller Brachytherapiebestrahlungsgeräte durchgeführt werden. Bei solchen Brachytherapieverfahren werden besagte Brachytherapiebestrahlungsgeräte dazu verwendet, Strahlung, insbesondere Röntgenstrahlung, im Inneren eines menschlichen oder tierischen Körpers in der Nähe von zu therapierendem Gewebe zu emittieren, um hierdurch das Gewebe zu bestrahlen.

Ein beispielhaftes Brachytherapiebestrahlungsgerät umfasst ein Partikelstrahlsystem, welches einen hochenergetischen Partikelstrahl erzeugen kann. Der Partikelstrahl wird durch ein einige Zentimeter langes Rohr des Brachytherapiebestrahlungsgerätes auf ein Röntgen-Material gerichtet, welches am Ende des Rohres angeordnet ist. Durch Wechselwirkung des Partikelstrahls mit dem Röntgen-Material erzeugt dieses Röntgenstrahlung, welche zur Bestrahlung des Gewebes vorgesehen ist.

Damit die von dem Röntgen-Material an dem Ende des Rohres erzeugte Röntgenstrahlung im Inneren eines Körpers angewendet werden kann, wird das Rohr in den Körper eingeführt. Hierzu ist das Rohr von einem auf das Brachytherapiebestrahlungsgerät aufsetzbaren starren Applikator umgeben, welcher einerseits eine sterile Barriere darstellt und andererseits das Rohr des Brachytherapiebestrahlungsgerätes schützt.

Eine Bestrahlung mittels eines solchen Brachytherapiebestrahlungsgerätes wird üblicherweise nach einer Resektion eines Tumors durchgeführt. Durch die Resektion entsteht in dem operierten Körper eine Kavität an der Stelle, an welcher zuvor der Tumor lag. Form und Größe der Kavität sind von Fall zu Fall verschieden und hängen im Wesentlichen von der Form und Größe des entfernten Tumors ab. Für eine effiziente Bestrahlung ist es vorteilhaft, wenn Form und Größe des verwendeten Applikators der Form und Größe der Kavität entsprechen.

Da sich die Kavität im Inneren des Körpers befindet und daher von außen nicht oder nur unzureichend einsehbar ist, ist die Bestimmung eines geeigneten Applikators in der Praxis schwierig. Herkömmlicherweise wird der geeignete Applikator durch Ausprobieren ermittelt.

Es ist daher eine Aufgabe der vorliegenden Erfindung, den Vorgang des Auswählens eines Applikators für eine Brachytherapiebestrahlungsgerät, welcher für die Bestrahlung eines eine Kavität umgebenden Gegenstands verwendet wird, zu verbessern.

Die Aufgabe wird gelöst durch eine Brachytherapievorrichtung und ein Brachytherapieverfahren gemäß den beigefügten Ansprüchen.

Insbesondere wird die Aufgabe gelöst durch eine Brachytherapievorrichtung, welche umfasst: ein stabförmiges Hauptelement, dessen Form eine Längsrichtung definiert; eine an dem Hauptelement montierte erste Halterung; eine an dem Hauptelement montierte zweite Halterung; mindestens ein sich zwischen der ersten Halterung und der zweiten Halterung ersteckendes flexibles Nebenelement, welches mit der ersten Halterung und der zweiten Halterung so verbunden ist, dass eine Änderung der Lage der ersten Halterung relativ zu der zweiten Halterung eine Änderung eines Abstands zwischen dem Hauptelement und dem mindestens einen Nebenelement, gemessen entlang einer zu der Längsrichtung orthogonal orientierten Querrichtung, bewirkt; und eine Messvorrichtung, welche dazu konfiguriert ist, basierend auf mindestens einer Koordinate der Lage der ersten Halterung relativ zu der zweiten Halterung einen Messwert anzugeben, welcher eine von dem Abstand abhängige Größe repräsentiert.

Ohne Beschränkung der Allgemeinheit wird nachfolgend davon ausgegangen, dass die Brachytherapievorrichtung mehrere Nebenelemente umfasst. Dies dient lediglich zur einfacheren Beschreibung. Die Brachytherapievorrichtung benötigt jedoch lediglich mindestens ein Nebenelement.

Die erste Halterung ist an dem Hauptelement montiert. Die erste Halterung kann fest oder bewegbar an dem Hauptelement montiert sein. Wenn die erste Halterung fest an dem Hauptelement montiert ist, kann die erste Halterung relativ zu dem Hauptelement nicht bewegt werden. Wenn die erste Halterung an dem Hauptelement bewegbar montiert ist, kann die erste Halterung relativ zu dem Hauptelement bewegt werden, beispielsweise entlang der Längsrichtung verschoben oder um die Längsrichtung gedreht werden. Weitere und andere Freiheitsgrade der Bewegung können vorgesehen sein.

Die zweite Halterung ist bewegbar an dem Hauptelement montiert. Das bedeutet, dass die zweite Halterung relativ zu dem Hauptelement bewegt werden kann, beispielsweise entlang der Längsrichtung verschoben oder um die Längsrichtung gedreht werden kann. Weitere und andere Freiheitsgrade der Bewegung können vorgesehen sein.

Die erste Halterung und die zweite Halterung sind an dem Hauptelement so montiert, dass die erste Halterung und die zweite Halterung zueinander gemäß mindestens einem Bewegungsfreiheitsgrad bewegbar sind. Beispielsweise sind die erste Halterung und die zweite Halterung an dem Hauptelement so montiert, dass die erste Halterung und die zweite Halterung relativ zueinander entlang der Längsrichtung bewegt werden können. Dies wird beispielsweise erreicht, indem die erste Halterung fest an dem Hauptelement montiert ist, während die zweite Halterung an dem Hauptelement entlang der Längsrichtung verschiebbar montiert ist. Dementsprechend ist die Position der zweiten Halterung entlang der Längsrichtung variierbar. Ebenso ist der Abstand entlang der Längsrichtung zwischen der ersten und zweiten Halterung variierbar.

Die erste Halterung, die zweite Halterung und die Nebenelemente sind funktionell, insbesondere mechanisch, miteinander so verbunden, dass eine Bewegung der ersten Halterung relativ zu der zweiten Halterung gemäß dem mindestens einen Bewegungsfreiheitsgrad (d. h. eine Änderung der Lage der ersten Halterung relativ zu der zweiten Halterung gemäß dem mindestens einen Bewegungsfreiheitsgrad) bewirkt, dass sich der Abstand zwischen dem Hauptelement und den Nebenelementen und/oder der Abstand zwischen den Nebenelementen ändert. Dementsprechend bewirkt eine Änderung der Lage der ersten Halterung relativ zu der zweiten Halterung gemäß dem mindestens einen Bewegungsfreiheitsgrad eine Änderung der in der Querrichtung gemessenen Breite der Brachytherapievorrichtung. Zur Vereinfachung der Beschreibung wird die Lage der ersten Halterung relativ zu der zweiten Halterung auch als Relativlage bezeichnet. Somit können durch Änderung der Relativlage besagter Abstand und besagte Breite verändert werden.

Die Messvorrichtung dient dazu, einen Messwert anzugeben, welcher eine von dem Abstand abhängige Größe repräsentiert. Eine Größe ist von dem Abstand abhängig, wenn eine Änderung des Abstands zu einer Änderung des Werts der Größe führt. Beispielsweise kann der Messwert den Abstand selbst repräsentieren. Der Messwert kann jedoch auch besagte Breite der Brachytherapievorrichtung repräsentieren, da die Breite der Brachytherapievorrichtung eine Größe ist, die von dem Abstand abhängig ist. Alternativ kann der Messwert eine Volumen-, Flächen- oder Längengröße repräsentieren, die von dem Abstand abhängig ist. Beispielsweise repräsentiert der Messwert ein Volumen, eine Querschnittsfläche oder einen Durchmesser einer imaginären Kugel, die durch die Nebenelemente approximiert wird.

Die Messvorrichtung gibt den Messwert in Abhängigkeit (d. h. auf Grundlage) mindestens einer Koordinate der Relativlage an, deren Änderung eine Änderung des Abstands zwischen dem Hauptelement und den Nebenelementen bewirkt (d. h. der Abstand zwischen dem Hauptelement und den Nebenelementen ist von der mindestens einen Koordinate der Relativlage abhängig). Eine Lage bzw. eine Relativlage zeichnet sich durch sechs Koordinaten aus, nämlich drei Koordinaten für die Position und drei Koordinaten für die Orientierung. Besagte mindestens eine Koordinate der Relativlage dient der Messvorrichtung als Grundlage, anhand welcher der Messwert angegeben wird. Besagte mindestens eine Koordinate der Relativlage kann eine beliebige Auswahl der sechs Koordinaten sein, wobei der Abstand zwischen dem Hauptelement und den Nebenelementen von mindestens einer der ausgewählten Koordinaten abhängig ist.

Beispielsweise kann die mindestens eine Koordinate der Relativlage die Position der ersten oder zweiten Halterung in Längsrichtung sein. Alternativ kann die mindestens eine Koordinate der Relativlage der Abstand zwischen der ersten und zweiten Halterung entlang der Längsrichtung sein. Weiter alternativ kann die mindestens eine Koordinate der Relativlage der Drehwinkel um die Längsrichtung zwischen der ersten Halterung und der zweiten Halterung sein.

Die Anwendbarkeit der vorangehend beschriebenen Brachytherapievorrichtung wird im Zusammenhang mit dem nachfolgend beschriebenen Brachytherapieverfahren deutlich. Das Verfahren setzt voraus, dass eine Kavität in einem Gegenstand, insbesondere in einem menschlichen oder tierischen Körper, vorhanden ist, deren umliegendes Gewebe mittels Strahlung bestrahlt werden soll.

Zunächst wird die Größe der Kavität unter Verwendung der Brachytherapievorrichtung bestimmt. Hierzu wird zuerst die Brachytherapievorrichtung in die Kavität eingeführt. Zum Einführen der Brachytherapievorrichtung in die Kavität ist es vorteilhaft, wenn die Brachytherapievorrichtung eine möglichst geringe Breite in Querrichtung aufweist. Dies kann dadurch erreicht werden, indem die Relativlage so angepasst wird, dass der Abstand zwischen dem Hauptelement und den Nebenelementen bzw. die davon abhängige Breite der Brachytherapievorrichtung minimiert oder zumindest reduziert ist.

Sobald die Brachytherapievorrichtung in der Kavität angeordnet ist, wird die Relativlage so geändert, dass der Abstand zwischen dem Hauptelement und den Nebenelementen bzw. die Breite der Brachytherapievorrichtung zunimmt. Die Relativlage wird auf diese Weise so lange geändert, bis die Brachytherapievorrichtung eine Breite angenommen hat, die so groß ist, dass sie der Breite der Kavität entlang der Querrichtung entspricht. In diesem Zustand entspricht die Breite der Brachytherapievorrichtung annähernd der Breite der Kavität. Somit ist die Breite der Brachytherapievorrichtung bzw. der Abstand zwischen dem Hauptelement und den Nebenelementen ein Maß für die Größe der Kavität.

Dieses Maß für die Größe der Kavität wird durch den Messwert repräsentiert, der von der Messvorrichtung angegeben wird. Somit kann der von der Messvorrichtung angegebene Messwert die Größe der Kavität repräsentieren. Durch Erfassen des Messwerts kann die Größe der Kavität bestimmt werden.

Nach dem Erfassen des Messwerts kann die Relativlage erneut geändert werden und zwar so, dass die Breite der Brachytherapievorrichtung minimiert oder zumindest reduziert ist. Somit kann die Brachytherapievorrichtung einfach aus der Kavität entfernt werden.

Für die nun folgende Bestrahlung des die Kavität umgebenden Gewebes wird nun in Abhängigkeit der unter Verwendung der Brachytherapievorrichtung bestimmten Größe der Kavität ein passender Applikator ausgewählt. Der so ausgewählte Applikator wird auf ein entsprechendes Brachytherapiebestrahlungsgerät aufgesetzt. Das mit dem ausgewählten Applikator versehene Brachytherapiebestrahlungsgerät wird nun in die Kavität eingeführt. Anschließend wird die Bestrahlung durchgeführt.

Ein wesentlicher Vorteil dieses Verfahrens ist, dass eine geeignete Größe für den Applikator einfach und schnell mittels der Brachytherapievorrichtung bestimmt werden kann. Es ist daher nicht erforderlich, die geeignete Größe des Applikators durch Ausprobieren oder unpräzise Abschätzungsverfahren zu bestimmen.

Ausführungsformen der Erfindung werden nachfolgend anhand von Figuren näher erläutert.
- Figuren 1A bis 1C: zeigen eine schematische Darstellung einer Brachytherapievorrichtung für verschiedene Relativlagen.
- Figur 2: zeigt eine schematische Darstellung einer weiteren Brachytherapievorrichtung.
- Figuren 3A bis 3C: zeigen schematisch dargestellte Schritte eines Brachytherapieverfahrens unter Verwendung der in den Figuren 1A bis 1C gezeigten Brachytherapievorrichtung.

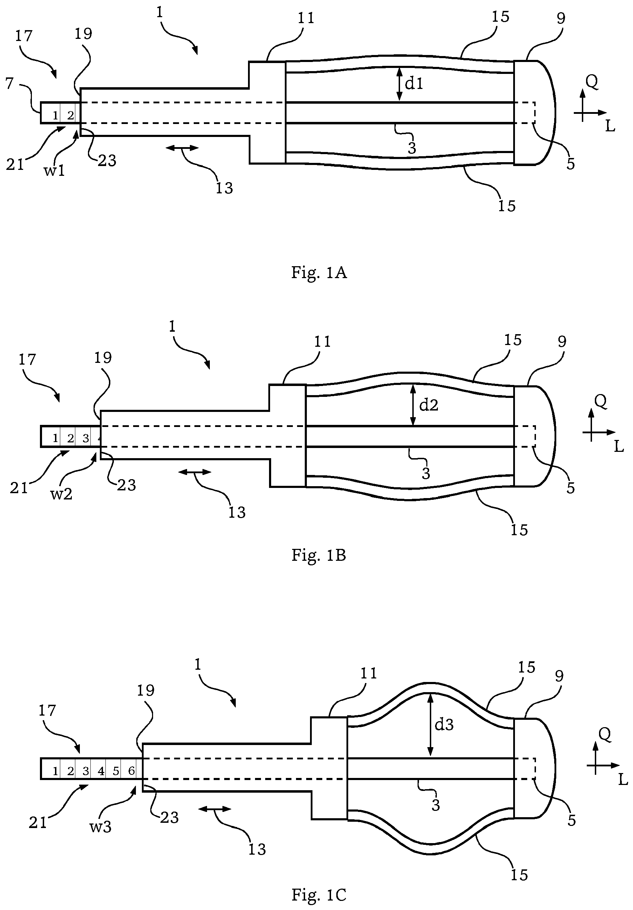

Eine beispielhafte Brachytherapievorrichtung 1 wird nachfolgend mit Bezug zu den Figuren 1A bis 1C beschrieben. Die Figuren 1A bis 1C zeigen eine Brachytherapievorrichtung 1 im Querschnitt in einer Ebene, die durch eine Längsrichtung L und eine Querrichtung Q aufgespannt wird. Die Querrichtung Q ist orthogonal zu der Längsrichtung L orientiert.

Die Brachytherapievorrichtung 1 umfasst ein stabförmiges Hauptelement 3 mit einem vorderen Ende 5 und einem hinteren Ende 7. Die Längsrichtung läuft geradlinig durch das vordere Ende 5 und das hintere Ende 7 des Hauptelements 3. Das Hauptelement 3 kann beispielsweise einen kreisförmigen oder quadratischen Querschnitt in einer Ebene aufweisen, deren Normale die Längsrichtung L ist. Entlang der Längsrichtung L kann das Hauptelement 3 eine Länge in der Größenordnung von beispielsweise 15 cm bis 40 cm aufweisen. Entlang der Querrichtung Q kann das Hauptelement 3 eine Breite in der Größenordnung von beispielsweise 3 mm bis 10 mm aufweisen. Die angegebenen Zahlenwerte dienen lediglich der Angabe einer geeigneten Größenordnung. Das Hauptelement 3 kann steif sein, d. h. nicht flexibel und nicht dehnbar sein.

Die Brachytherapievorrichtung 1 umfasst ferner eine erste Halterung 9, welche an dem Hauptelement 3 montiert ist. In dem in den Figuren gezeigten Beispiel ist die erste Halterung 9 fest an dem vorderen Ende 5 des Hauptelements 3 montiert, so dass die erste Halterung 9 relativ zu dem Hauptelement 3 nicht bewegt werden kann. Beispielsweise ist die erste Halterung 9 mit dem Hauptelement 3 durch eine Klebe- oder Schraubverbindung verbunden.

Die Brachytherapievorrichtung 1 umfasst ferner eine zweite Halterung 11, welche an dem Hauptelement 3 montiert ist. In dem in den Figuren gezeigten Beispiel ist die zweite Halterung 11 an dem Hauptelement 3 bewegbar montiert. Genauer gesagt ist die zweite Halterung 11 relativ zu dem Hauptelement 3 entlang der Längsrichtung L verschiebbar, was durch den Doppelpfeil 13 in den Figuren gekennzeichnet ist. Dies kann beispielsweise dadurch erreicht werden, dass das Hauptelement 3 die zweite Halterung 11 in einer Ausnehmung durchsetzt, die in der zweiten Halterung 11 gebildet ist. In dem mit Bezug zu den Figuren 1A bis 1C erläuterten Beispiel dient die Position der zweiten Halterung 11 entlang der Längsrichtung L stellvertretend für die allgemeinere Lehre Relativlage.

Die Brachytherapievorrichtung 1 umfasst ferner zwei Nebenelemente 15. Beispielsweise sind die Nebenelemente 15 flexible aber nicht dehnbare Streifen oder Stäbe. Die Nebenelemente 15 erstrecken sich jeweils zwischen der ersten Halterung 9 und der zweiten Halterung 11. Die Nebenelemente 15 sind um das Hauptelement 3 herum angeordnet. Jedes der Nebenelemente 15 ist sowohl mit der ersten Halterung 9 als auch mit der zweiten Halterung 11 verbunden. Die Nebenelemente 15 können an der ersten Halterung 9 und der zweiten Halterung 11 auf beliebige Weise befestigt sein, beispielsweise durch eine formschlüssige Verbindung, eine Klebeverbindung oder dergleichen. Die Nebenelemente 15 können an der ersten Halterung 9 und/oder der zweiten Halterung 11 verstellbar befestigt sein, sodass die Länge der Nebenelemente 15 zwischen der ersten Halterung 9 und der zweiten Halterung 11 variabel einstellbar ist. Die Nebenelemente 15 können starr oder flexibel (beispielsweise durch Gelenke) mit der ersten Halterung 9 und/oder der zweiten Halterung 11 verbunden sein. Die Brachytherapievorrichtung 1 kann mehr als zwei Nebenelemente 15 dieser Art umfassen.

Die Nebenelemente 15 sind an der ersten Halterung 9 und der zweiten Halterung 11 so angebracht, dass eine Änderung der Position der zweiten Halterung 11 entlang der Längsrichtung L (stellvertretend für die allgemeinere Lehre der Änderung der Relativlage gemäß einem Bewegungsfreiheitsgrad) eine Änderung eines Abstands zwischen dem Hauptelement 3 und den Nebenelementen 15 entlang der Querrichtung Q bewirkt.

Figur 1A zeigt die Brachytherapievorrichtung 1 in einem ersten Zustand, in welchem die zweite Halterung 11 relativ weit von der ersten Halterung 9 entlang der Längsrichtung L entfernt ist. Bei dieser Position der zweiten Halterung 11 entlang der Längsrichtung L weist der Abstand zwischen dem Hauptelement 3 und den Nebenelementen 15 den Wert d1 auf.

Figur 1B zeigt die Brachytherapievorrichtung 1 in einem zweiten Zustand. Im Vergleich zu dem in Figur 1A gezeigten ersten Zustand ist die zweite Halterung 11 näher an der ersten Halterung 9 angeordnet, was beispielsweise durch Bewegen der zweiten Halterung 11 entlang der Längsrichtung L in Richtung der ersten Halterung 9 erreicht werden kann. Durch das Bewegen der zweiten Halterung 11 hin zu der ersten Halterung 9 (entspricht Änderung der Relativlage gemäß einem Bewegungsfreiheitsgrad) wird in den Nebenelementen 15 eine Spannung erzeugt, die bewirkt, dass sich die Nebenelemente 15 verformen, da die Nebenelemente 15 flexibel und nicht dehnbar sind. Konkret werden die Nebenelemente 15 von dem Hauptelement 3 weg nach außen gekrümmt, wodurch der Abstand zwischen dem Hauptelement 3 und den Nebenelementen 15, gemessen in der Querrichtung Q, von dem Wert d1 (siehe Figur 1A) auf den Wert d2 (siehe Figur 1B) zunimmt. Damit hat auch die Breite der Brachytherapievorrichtung 1, gemessen in der Querrichtung Q, zugenommen.

Figur 1C zeigt die Brachytherapievorrichtung 1 in einem dritten Zustand. Im Vergleich zu dem in Figur 1B gezeigten zweiten Zustand ist die zweite Halterung 11 noch näher an der ersten Halterung 9 angeordnet, was beispielsweise durch Bewegen der zweiten Halterung 11 entlang der Längsrichtung L in Richtung der ersten Halterung 9 erreicht werden kann. Die Nebenelemente 15 weisen daher einen noch größeren Wert d3 für den Abstand von dem Hauptelement 3 auf. Damit hat auch die Breite der Brachytherapievorrichtung 1, gemessen in der Querrichtung Q, nochmals zugenommen.

Die Brachytherapievorrichtung 1 umfasst ferner eine Messvorrichtung 17, welche dazu konfiguriert ist, basierend auf der Position der zweiten Halterung 11 entlang der Längsrichtung L (stellvertretend für die allgemeinere Lehre der mindestens einen Koordinate der Relativlage) einen Messwert w anzugeben, welcher eine von dem Abstand zwischen dem Hauptelement 3 und den Nebenelementen 15 abhängige Größe repräsentiert.

In dem in den Figuren 1A bis 1C gezeigten Beispiel umfasst die Messvorrichtung 17 eine Ablesemarkierung 19 und eine Skala 21. Die Ablesemarkierung 19 wird durch ein hinteres Ende 23 der zweiten Halterung 11 gebildet. Die Skala 21 befindet sich auf der Oberfläche des Hauptelements 3 und besteht beispielsweise aus einer Reihe von Markierungen (z. B. Striche) und einer Reihe von Beschriftungen (z. B. Zahlen), die den Markierungen zugeordnet sind. Die Position der Ablesemarkierung 19 über der Skala 21 hängt von der Position der zweiten Halterung 11 entlang der Längsrichtung L ab. Das bedeutet, dass eine Änderung der Position der zweiten Halterung 11 entlang der Längsrichtung L zu einer Änderung der Position der Ablesemarkierung 19 über der Skala 21 führt. Die Ablesemarkierung 19 gibt den Messwert auf der Skala 21 an.

Figur 1A zeigt die Brachytherapievorrichtung 1 in dem ersten Zustand. Die Lage der ersten Halterung 9 relativ zu der zweiten Halterung 11, vorliegend repräsentiert durch die Position der zweiten Halterung 11 entlang der Längsrichtung L, bewirkt den Abstand d1 zwischen den Nebenelementen 15 und dem Hauptelement 3. Die Messvorrichtung 17 gibt für diese Position der zweiten Halterung 11 entlang der Längsrichtung L einen Messwert w1 an, der in Figur 1A näherungsweise den Wert 2 aufweist. Der Messwert w1 repräsentiert eine Größe, die von dem Abstand d1 abhängig ist. Beispielsweise repräsentiert der Messwert w1 die Breite der Brachytherapievorrichtung 1 entlang der Querrichtung Q in der Einheit Zentimeter. Der Messwert kann jedoch jede beliebig definierte Größe repräsentieren, die von dem Abstand zwischen den Nebenelementen 15 und dem Hauptelement 3 abhängig ist (das heißt jede Größe, deren Wert sich ändert, wenn der Wert des Abstands variiert).

Figur 1B zeigt die Brachytherapievorrichtung 1 in dem zweiten Zustand, in welchem sich durch Verlagerung der zweiten Halterung 11 hin zu der ersten Halterung 9 die Position der zweiten Halterung 11 entlang der Längsrichtung L geändert hat, wodurch der Abstand zwischen den Nebenelementen 15 und dem Hauptelement 3, gemessen in der Querrichtung Q, den Wert d2 aufweist, der größer als der Wert d1 ist. Dementsprechend gibt die Messvorrichtung 17 nun einen anderen Messwert an, nämlich den Messwert w2, der annähernd den Wert 4 hat.

Figur 1C zeigt die Brachytherapievorrichtung 1 in dem dritten Zustand, in welchem sich durch weitere Verlagerung der zweiten Halterung 11 hin zu der ersten Halterung 9 die Position der zweiten Halterung 11 entlang der Längsrichtung L nochmals geändert hat. Dementsprechend hat sich auch der Abstand zwischen den Nebenelementen 15 und dem Hauptelement 3, gemessen in der Querrichtung Q, auf den Wert d3 geändert, der größer als der Wert d2 ist. Dementsprechend gibt die Messvorrichtung 17 nun erneut einen anderen Messwert an, nämlich den Messwert w3, der annähernd den Wert 6 hat.

Die Ausgestaltung der Skala definiert die Bedeutung des Messwerts. Nach Definition der Bedeutung des Messwerts, beispielsweise als die Breite der Brachytherapievorrichtung 1 entlang der Querrichtung Q, kann die Skala beispielsweise experimentell bestimmt werden.

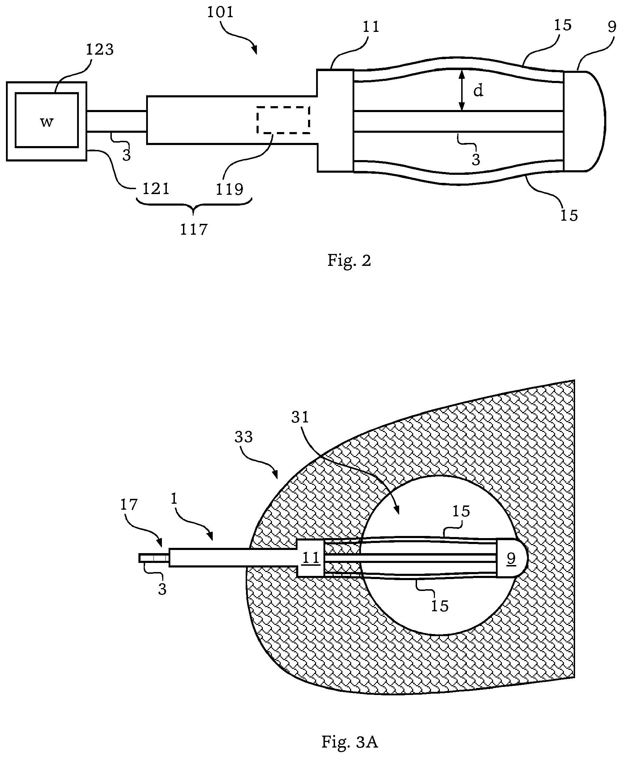

Figur 2 zeigt eine schematische Darstellung einer weiteren Brachytherapievorrichtung 101. Die Brachytherapievorrichtung 101 entspricht weitgehend der Brachytherapievorrichtung 1, die vorangehend mit Bezug zu den Figuren 1A bis 1C beschrieben wurde. Der wesentliche Unterschied zwischen der Brachytherapievorrichtung 101 der Figur 2 und der Brachytherapievorrichtung 1 der Figuren 1A bis 1C besteht in der Ausgestaltung der Messvorrichtung 117. Die Messvorrichtung 117 umfasst eine Sensorvorrichtung 119, welche dazu konfiguriert ist, die Position der zweiten Halterung 11 entlang der Längsrichtung L (stellvertretend für die allgemeinere Lehre der mindestens einen Koordinate der Relativlage) zu messen. Für die Bestimmung der Position der zweiten Halterung 11 entlang der Längsrichtung L sind dem Fachmann zahlreiche Sensorvorrichtungen bekannt. Beispielsweise kann die Position mittels Messung des Abstands zwischen der zweiten Halterung 11 und der ersten Halterung 9 mittels Interferenzmessung durchgeführt werden. Alternativ kann das Hauptelement 3 eine entlang der Längsrichtung orientierte Zahnstange umfassen und die zweite Halterung 11 weist ein in die Zahnstange eingreifendes Zahnrad auf, wobei die Drehstellung des Zahnrads gemessen wird und basierend darauf die Position der zweiten Halterung 11 bestimmt wird.

Die Messvorrichtung 117 umfasst ferner eine Steuerung 121, welche dazu konfiguriert ist, basierend auf der mittels der Sensorvorrichtung 119 bestimmten Position der zweiten Halterung 11 entlang der Längsrichtung L den Messwert w zu bestimmen und auszugeben. Zum Ausgeben des Messwerts w umfasst die Messvorrichtung 117 eine Anzeige 123, welche den Messwert w anzeigen kann. Die Steuerung 121 und die Anzeige 123 sind am hinteren Ende des Hauptelements 3 angeordnet.

Die vorangehend beschriebenen Messvorrichtungen 17 und 117 sind lediglich Beispiele für mögliche Ausgestaltungen der Messvorrichtung.

Nachfolgend wird mit Bezug zu den Figuren 3A bis 3C ein Brachytherapieverfahren erläutert.

Figur 3A zeigt eine schematische Darstellung einer Kavität 31 innerhalb eines Gegenstands 33 im Querschnitt. Der Gegenstand 33 kann beispielsweise ein menschlicher oder tierischer Körper sein. Durch eine in den Figuren 3A bis 3C nicht dargestellte Öffnung wurde eine Brachytherapievorrichtung 1 (stellvertretend für jegliche hierin beschriebenen Brachytherapievorrichtungen) in die Kavität 31 eingeführt. Zur Vereinfachung des Einführens in die Kavität 31 wurde die Relativlage so eingestellt, dass die Breite der Brachytherapievorrichtung 1 möglichst gering ist, so wie dies in Figur 3A dargestellt ist.

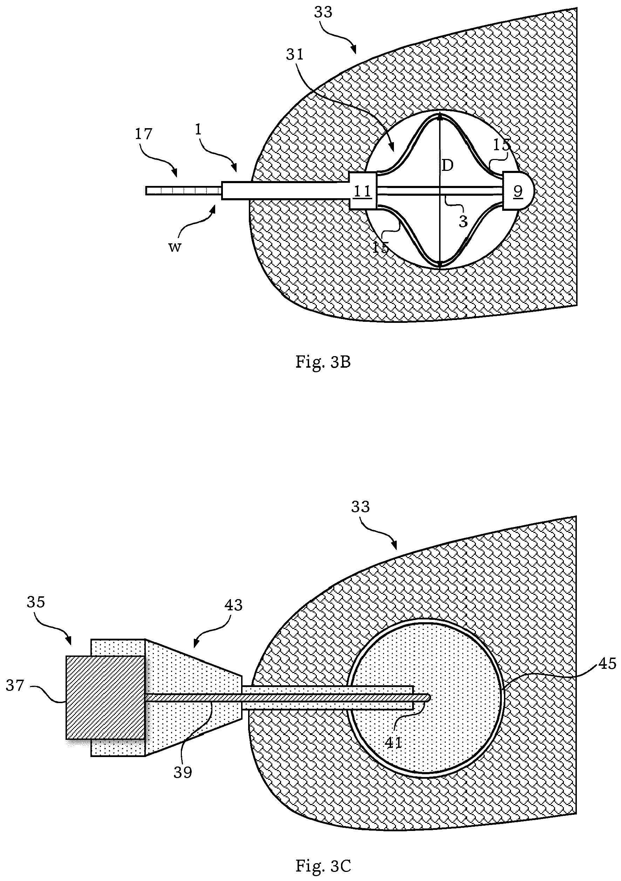

Sobald die Brachytherapievorrichtung 1 in die Kavität 31 eingeführt ist, wird unter Verwendung der Brachytherapievorrichtung 1 die Größe D der Kavität 31 bestimmt. Wie in Figur 3B dargestellt, wird hierzu die Relativlage so geändert, dass die Nebenelemente 15 an die Oberfläche der Kavität 31 heranreichen. Hierdurch approximieren die Nebenelemente 15 die Größe der Kavität 31. In dieser Situation ist der auf der Skala 21 ablesbare Messwert (stellvertretend für die allgemeine Lehre des von der Messvorrichtung angegebenen Messwerts) ein Maß für die Größe D der Kavität 31. Somit kann die Größe D der Kavität 31 unter Verwendung der Brachytherapievorrichtung 1 bestimmt werden.

Danach kann die Relativlage wieder so geändert werden, dass die in Figur 3A gezeigte Situation entsteht, in welcher die Breite der Brachytherapievorrichtung 1 minimiert ist, und die Brachytherapievorrichtung 1 kann durch die nicht dargestellte Öffnung aus der Kavität 31 entfernt werden.

Anschließend kann der Gegenstand 33 lokal im Bereich der Kavität 31 unter Verwendung eines Brachytherapiebestrahlungsgerätes 35 bestrahlt werden. In Figur 3C ist ein solches Brachytherapiebestrahlungsgerät 35 dargestellt.

Das Brachytherapiebestrahlungsgerät 35 umfasst in diesem Beispiel einen Hauptkörper 37 und ein Rohr 39, an dessen Ende ein Röntgen-Material 41 angeordnet ist. Innerhalb des Hauptkörpers 37 kann ein hochenergetischer Partikelstrahl erzeugt werden, der durch das Rohr 39 auf das Röntgen-Material 41 gerichtet wird, wodurch Röntgenstrahlung erzeugt wird.

Zur Erzeugung eines vordefinierten Strahlungsprofils und zum Schutz des Brachytherapiebestrahlungsgerätes 35 wird vor der Bestrahlung ein Applikator 43 auf das Brachytherapiebestrahlungsgerät 35 aufgesetzt. Der Applikator 43 umgibt das Rohr 39 und weist an seinem vorderen Ende einen Applikatorkopf 45 auf, der beispielsweise eine kugelförmige Außenform aufweist. Der Applikator 43 wird üblicherweise aus einem starren Material gefertigt und weist daher eine starre Außenform auf. Daher ist es erforderlich, Form und Größe des Applikatorkopfes 45 so zu wählen, dass sie zu der Form und Größe der Kavität 31 passen. Da die Größe D der Kavität 31 bereits unter Verwendung der Brachytherapievorrichtung 1 bestimmt wurde, kann basierend darauf ein geeigneter Applikator 43 einfach ausgewählt werden. Der auf diese Weise ausgewählte Applikator 43 kann sodann auf das Brachytherapiebestrahlungsgerät 35 aufgesetzt werden und mit diesem zur Bestrahlung der die Kavität 31 umgebenden Gebiete des Gegenstands 33 verwendet werden.

Die vorangehend beschriebene Brachytherapievorrichtung ermöglicht eine einfache Bestimmung der Größe einer Kavität. Hierdurch wird die Auswahl eines geeigneten Applikators vereinfacht und eine fehlerhafte Auswahl kann vermieden werden.

## Patentansprüche

1. Brachytherapievorrichtung (1, 101), umfassend:
ein stabförmiges Hauptelement (3), dessen Form eine Längsrichtung (L) definiert;
eine an dem Hauptelement (3) montierte erste Halterung (9);
eine an dem Hauptelement (3) montierte zweite Halterung (11);
mindestens ein sich zwischen der ersten Halterung (9) und der zweiten Halterung (11) ersteckendes flexibles Nebenelement (15), welches mit der ersten Halterung (9) und der zweiten Halterung (11) so verbunden ist, dass eine Änderung der Lage der ersten Halterung (9) relativ zu der zweiten Halterung (11) eine Änderung eines Abstands (d) zwischen dem Hauptelement (3) und dem mindestens einen Nebenelement (15), gemessen entlang einer zu der Längsrichtung (L) orthogonal orientierten Querrichtung (Q), bewirkt; und
eine Messvorrichtung (17, 117), welche dazu konfiguriert ist, basierend auf mindestens einer Koordinate der Lage der ersten Halterung (9) relativ zu der zweiten Halterung (11) einen Messwert (w) anzugeben, welcher eine von dem Abstand (d) abhängige Größe repräsentiert.

2. Brachytherapievorrichtung (1) nach Anspruch 1, wobei die Messvorrichtung (17) eine Ablesemarkierung (19) und eine Skala (21) umfasst, wobei eine Position der Ablesemarkierung (19) auf der Skala (21) von der mindestens einen Koordinate der Lage der ersten Halterung (9) relativ zu der zweiten Halterung (11) abhängig ist und wobei die Ablesemarkierung (19) den Messwert (w) auf der Skala (21) angibt.

3. Brachytherapievorrichtung (101) nach Anspruch 1 oder 2, wobei die Messvorrichtung (117) eine Sensorvorrichtung (119) umfasst, welche dazu konfiguriert ist, die mindestens eine Koordinate der Lage der ersten Halterung (9) relativ zu der zweiten Halterung (11) zu messen; und
wobei die Messvorrichtung (117) ferner eine Steuerung (121) umfasst, welche dazu konfiguriert ist, basierend auf der mittels der Sensorvorrichtung (119) bestimmten mindestens einen Koordinate den Messwert (w) zu bestimmen und auszugeben.

4. Brachytherapievorrichtung (1, 101) nach einem der Ansprüche 1 bis 3, wobei der Messwert (w) eine Volumen-, Flächen- oder Längengröße repräsentiert, die sich in Abhängigkeit des Abstands (d) zwischen dem Hauptelement (3) und dem mindestens einen Nebenelement (15) ändert.

5. Brachytherapievorrichtung (1, 101) nach einem der Ansprüche 1 bis 4, wobei der Messwert (w) ein Volumen, eine Querschnittsfläche oder einen Durchmesser einer imaginären Kugel repräsentiert, die durch das mindestens eine Nebenelement (15) approximiert wird.

6. Brachytherapievorrichtung (1, 101) nach einem der Ansprüche 1 bis 5,
wobei das Hauptelement (3) steif ist und/oder
wobei das mindestens eine Nebenelement (15) nicht dehnbar ist.

7. Brachytherapievorrichtung (1, 101) nach einem der Ansprüche 1 bis 6, welches eine Vielzahl von sich zwischen der ersten Halterung (9) und der zweiten Halterung (11) ersteckenden flexiblen Nebenelementen (15) umfasst, wobei jedes der Nebenelemente (15) mit der ersten Halterung (9) und der zweiten Halterung (11) so verbunden ist, dass eine Änderung der Lage der ersten Halterung (9) relativ zu der zweiten Halterung (11) eine Änderung eines Abstands (d) zwischen dem Hauptelement (3) und dem jeweiligen Nebenelement (15), gemessen entlang der Querrichtung (Q), bewirkt.

8. Brachytherapievorrichtung (1, 101) nach Anspruch 7, wobei die Nebenelemente (15) um das Hauptelement (3) herum angeordnet sind.

9. Brachytherapievorrichtung (1, 101) nach einem der Ansprüche 1 bis 8, wobei die erste Halterung (9) fest an dem Hauptelement (3) montiert ist und die zweite Halterung (11) relativ zu dem Hauptelement (3) bewegbar ist, insbesondere entlang der Längsrichtung (L) verschiebbar ist und/oder um die Längsrichtung (L) drehbar ist.

10. Brachytherapieverfahren, umfassend:
Bestimmen einer Größe (D) einer Kavität (31) unter Verwendung der Brachytherapievorrichtung (1, 101) nach einem der Ansprüche 1 bis 9;
Auswählen eines Applikators (43) für ein Brachytherapiebestrahlungsgerät (35) basierend auf der bestimmten Größe (D) der Kavität (31);
Verwenden des ausgewählten Applikators (43) zur Bestrahlung eines die Kavität umgebenden Gegenstands (3) mit dem Brachytherapiebestrahlungsgerät (35).

11. Brachytherapieverfahren nach Anspruch 10, wobei der Applikator (43) eine starre Außenform aufweist.
